# EUROPEAN PATENT APPLICATION

(11) **EP 2 644 693 A1**
(43) Date of publication of application: **02.10.2013**
(21) Application number: 12002306.4
(22) Date of filing: 29.03.2012
(51) Int. Cl.: C12N 1/20, C12N 5/10, C12N 15/70, C12N 15/09, C12N 9/18

(54) **Biologically active polypertide expressed on the surface of a cell**

(71) Applicant: IP Bewertungs AG (IPB), 20354 Hamburg (DE)
(72) Inventor: Coksezen, Atilla, Dr., 20146 Hamburg (DE); Rehn, Ulrike, Dr., 20535 Hamburg (DE); Richter, Carsten, Dr., 48143 Münster (DE); Kähler, Markus, Dr., 21075 Hamburg (DE); Stachelhaus, Torsten, Dr., 22417 Hamburg (DE)

(57) **Abstract**

The present invention is directed to a cell or multitude of cells expressing on its surface an activating polypeptide and/or a biologically active polypeptide, which biologically active polypeptide shows a biological activity subject to activation of the activating polypeptide. The present invention is also directly to a membrane preparation prepared from such a cell or multitude of cells, and a biologically active polypeptide, prepared from a cell or multitude of cells or a membrane preparation, and the use of such a cell or multitude of cells, membrane fraction, or polypeptide for producing a product of a reaction catalyzed by a biologically active polypeptide.

## Description

The present invention is directed to a cell or multitude of cells expressing on its surface an activating polypeptide and/or a biologically active polypeptide, which biologically active polypeptide shows a biological activity subject to activation of the activating polypeptide. The present invention is also directly to a membrane preparation prepared from such a cell or multitude of cells, and a biologically active polypeptide, prepared from a cell or multitude of cells or a membrane preparation, and the use of such a cell or multitude of cells, membrane fraction, or polypeptide for producing a product of a reaction catalyzed by a biologically active polypeptide.

Depending on the eventual intended use, polypeptides are required in different degrees of purity. While crude extracts may be used for some applications, however, polypeptides which are purified or at least enriched in that the sought-after activity is increased and other, potentially competitive activities are, to a certain degree at least, removed, will be required for many uses.

Polypeptide purification is usually a rather laborious and time-consuming procedure. Not only is a suitable source yielding sufficient an amount of starting material essential. A pellet of E. coli cells overexpressing the polypeptide of interest is in many cases the starting material of choice, wild type bacterial cell pellet or hard-to-process tissue material expressing the polypeptide at wild type levels are in some cases the only source accessible. However, there may be the need to go through multiple purification steps, each time-consuming and involving the use of expensive agents and materials, in order to receive the polypeptide in the required purity.

Even the purification of a single polypeptide may be a challenge, but this is all the more true if more than one polypeptide component is required. There are numerous examples of proteins showing industrially relevant and highly sought-after activities that require, following the ribosomal or non-ribosomal synthesis of the polypeptide chain, interaction of some sort with at least one other polypeptide in order for the first protein to be given a property or activity of interest. For example, many polypeptides require chemical modification, for example phosphorylation by a specific or unspecific phosphorylase, or chemical cleavage of a polypeptide chain by a protease, converting a precursor into an active polypeptide. Other polypeptides have a need to interact with chaperones assisting the folding of a fully or partially unfolded polypeptide chain into a protein of distinct three-dimensional structure.

Consequently, in order for such an activity of interest to be obtained at a large scale, considerable amounts of both polypeptides need to be purified, a reaction mixture comprising both polypeptides needs to be set up under conditions that promote activation by way of interaction. Finally, there may well be the need to remove the activating polypeptide, for example in case of a second protein showing a competitive reaction or to comply with regulatory standards. It must be borne in mind, in addition, that it is notoriously difficult to separate interacting polypeptides, as two such components usually associate to some extent, often forming extremely stable complexes.

Chaperone-dependent lipases from Pseudomonas and Burkholderia are a prime example of industrially relevant polypeptides that show a biological activity regulated by way of interaction with another polypeptides. Lipases (triacylglycerol hydrolases) are frequently used in organic synthesis (Kazlauskas and Bornscheuer, 1998; Schmid and Verger, 1998, Bornscheurs Buch). They are widely found in nature (animals, man, bacteria, yeast, fungi, plants) Lipases hydrolyse and reesterify triglycerides such as natural fats and oil in a reaction catalysed by a triad composed of Ser, His and Asp, similar to serine peptidases. The reaction comprises the reaction of the substrate with active-site serine, yielding a tetrahedral intermediate stabilised by the catalytic His- and Asp-residues, release of the alcohol, formation of a covalent acyl-enzyme complex, attack of a nucleophile such a water, whereby a tetrahedral intermediate is formed, and collapse of the intermediate to yield the product.

Industrially relevant reactions catalysed by lipases include the enantioselective acylation of (R,S)-1-phenethyl amino, the production of a Captopril intermediate, (R)-3-chloro-2-methly propionate, and the conversion and resolution of tertiary alcohols. Beside their use in organic synthetic chemistry, lipases are used in a variety of other areas. Applications include laundry detergents, cheese-making, modification of natural fats and oils (e.g. the synthesis of cocoa-butter equivalents, monoglycerides, in corporation of polyunsaturated fatty acids for human consumption), synthesis of sugar esters and simple esters used in personal care.

The main interest in the application of lipases in organic chemistry is owing to the fact that lipases are highly active in a broad range of non-aqueous solvents, often exhibit excellent stereoselectivity, accept a broad range of substrates as well as various nucleophiles such as water, alcohols and amines.

There is a multitude of lipases from various organisms, however, lipases of bacterial origins are particularly sought-after as potential host organisms, E. coli in particular, are easy to grow and amenable to genetic engineering techniques. Bacterial lipases fall in two groups: one group comprises lipases such as Bacillus subtilis lipase LipA, Fusarium solani pisi cutinase and Serratia marcescens lipase, which are reasonably robust enzymes that are straightforward to use. The second group comprises lipases from the genera Pseudomonas and Burkholderia which are of particular industrial interest as they catalyse a broad range of different reaction in water and non-polar solvents under mild conditions, but at same time, show a remarkable degree of stereoselectivity (1-4 aus Trodler et al.). Preparations of these lipases, in particular of Pseudomonas cepacia, are commercially available and have often been used by organic chemists for enantioselective syntheses, for example the enantioselective production of alcohols, amines and carboxylic acids (No 33 from Quyen et al., No1-6 aus Wilhelm et al). Unfortunately, these lipases require, in order for them to fold correctly, functional assistance of specific chaperones, referred to as "LIF" proteins (lipase specific foldases) (No 7 und 10 aus Wilhelm et al.). These LIF proteins are a unique family of steric chaperones anchored to the periplasmic side of the bacterial inner membrane in various Gram-negative bacteria (No 10-12 aus Wilhelm et al.).

Yang et al. (2010) have expressed chaperone-dependent lipases from Pseudomonas and Burkholderia on the surface of S. coli using the autotransporter system. The respective lipases were coexpressed, but not on the surface. Therefore, there was no interaction, let alone activation on the surface. Lee et al. (2005) expresses the Pseudomonas fluorescens SIK W1 lipase gene on the surface of Pseudomonase putida TK2442, but this is not a chaperone-dependent lipase. The same lipase was expressed on the surface of E. coli by Choi et al. (2004).

Therefore, the problem underlying the present invention is to provide the activity of a polypeptide that, in order for it to show activity, requires activation by another polypeptide, without the need to go through a laborious purification procedure. The activity should be specific to a certain degree, i.e. some sort of enrichment of the sought-after activity and separation of the polypeptide, and its activity, from unwanted components of the polypeptide-expressing cells, for instance cellular enzymes showing competitive activities, is paramount. Another problem underlying the present invention is to provide a method that allows for the production and/or preparation of the product of a protein that requires activation by another polypeptide. Another problem underlying the present invention is to provide the activity of such a polypeptide without having to purify the polypeptide capable of activating the first polypeptide repeatedly.

The present inventors have surprisingly found that a protein that requires activation by another protein may not only be expressed on the surface of cells, but may, when located on the surface of a cell, interact with other proteins to the effect that the first protein is activated. Moreover, the present inventors have surprisingly found that such activity may be obtained by way of interaction with another protein displayed on the surface of a cell. Even more surprisingly, such a protein activated on the surface of a cell may show, in terms of specificity and catalytic efficiency, properties compared to the protein expressed in the cytosol of an organism.

The problem underlying the present invention is solved by a cell or multitude of cells in form of a cell expressing on its surface an activating polypeptide and/or a biologically active polypeptide, which biologically active polypeptide shows a biological activity subject to activation by the activating polypeptide, wherein at least one of the activating polypeptide and the biologically active polypeptide is encoded by a nucleic acid comprising (1) a portion encoding a signal peptide (2) a portion encoding the biologically active polypeptide and/or the activating polypeptide (3) optionally a portion encoding a protease recognition site (4) optionally a portion encoding a transmembrane linker, and (5) optionally a portion encoding a transporter domain of an autotransporter; or multitude of cells comprising a first cell expressing on its surface an activating polypeptide and a second cell expressing on its surface a biologically active polypeptide, wherein at least one of the activating polypeptide and the biologically active polypeptide is encoded by a nucleic acid comprising (1) a portion encoding a signal peptide (2) a portion encoding the first polypeptide and/or the activating polypeptide (3) optionally a portion encoding a protease recognition site (4) optionally a portion encoding a transmembrane linker, and (5) optionally a portion encoding a transporter domain on an autotransporter.

In a preferred embodiment, the term "biologically active polypeptide", as used herein, refers to a polypeptide that affects some parameter of biological systems or chemical reactions, for example by altering growth of an organism, by affecting association of molecules and/or cells and by slowing down or accelerating chemical reactions. In a preferred embodiment, the biologically active polypeptide is an enzyme.

Accordingly, in one aspect the present invention is directed to a cell which expresses on its surface both an activating polypeptide and a biologically active polypeptide, which biologically active polypeptide shows a biological activity subject to activation by the activating polypeptide. In a second aspect, the present invention is directed to a multitude of cells, whereby a first cell expresses on its surface an activating polypeptide and a second cell expresses on its surface a biologically active polypeptide, which biologically active polypeptide shows a biological activity subject to activation by the activating polypeptide.

In a preferred embodiment, the cell expresses on its surface a fusion polypeptide comprising both the activating polypeptide and the biologically active polypeptide. The person skilled in the art is familiar with procedures, for instance overlap polymerase chain reactions, that may be used to fuse coding nucleic acid sequences, thus creating a sequence encoding a fusion protein, which sequence is then straightforward to express.

The present invention is also directed to a composition comprising a first cell expressing on its surface an activating polypeptide and a second cell expressing on its surface a biologically active polypeptide, wherein at least one of the activating polypeptide and the biologically active polypeptide is encoded by a nucleic acid comprising a portion encoding a signal peptide, a portion encoding the first polypeptide and/or the activating polypeptide, optionally a portion encoding a protease recognition site, optionally a portion encoding a transmembrane linker, and a portion encoding a transporter domain of an autotransporter. In a preferred embodiment, both the biologically active polypeptide and the activating polypeptide are encoded by such a nucleic acid.

Preferably, the biologically active polypeptide is activated by a chemical modification catalyzed by the activating polypeptide. In a preferred embodiment, the term "chemical modification", as used herein, refers to the formation and/or cleavage of at least one covalent bond.

It is further preferred that the biologically active polypeptide is activated by a conformational change brought about by a chaperone-like activity of the activating polypeptide.

In a preferred embodiment the biologically active polypeptide comprises a chaperone-dependent lipase and the activating polypeptide comprises a chaperone that recognizes as its substrate said chaperone-dependent lipase.

In a preferred embodiment, the biologically active polypeptide comprises a chaperone-dependent lipase and the activating polypeptide comprises a chaperone that recognizes as its substrate said chaperone-dependent lipase. In a preferred embodiment, the term "chaperone", as used herein, is a macromolecule, preferably a polypeptide, that alters the conformation of an other molecule, preferably a macromolecule, even more preferably a polypeptide or an RNA molecule. A chaperone may or may not have ATPase activity.

It is further preferred that the biologically active polypeptide and the activating polypeptide are independently selected from the polypeptides derived from the genera Pseudomonas, Burkholderia, Chromobacterium, Ralstonia, Acinetobacter, Vibrio and Xylella.

Preferably, the biologically active polypeptide is derived from Pseudomonas aeruginosa (accession no. D50587), Pseudomonas fragi (accession no. X14033), Pseudomonas luteola (accession no. AF050153), Pseudomonas wisconsinensis (accession no. U88907), Pseudomonas alcaligenes (accession no. CFR 708) Burkholderia cepacia (accession no. M58494), Burkholderia glumae (accession no. X70354), Chromobacterium viscosum (accession no. Q05489), Ralstonia metallidurans, Ralstonia solanacearum (accession no. AL 646081), Acinetobacter calcoaceticus (accession no. X80800), Vibrio cholerae (accession no. X16945), Vibrio vulnificus, Xylella fastidosa and variants thereof.

Preferably, the biologically active polypeptide is the chaperone-dependent lipase from Burkholderia cepacia ATCC 21808 or a variant thereof and the activating polypeptide is the chaperone activating the chaperone-dependent lipase from Burkholderia cepacia ATCC 21808 or a variant thereof.

In a preferred embodiment, the activating polypeptide is a foldase.

Lipases represent one of the most important classes of enzymes applied in organic chemistry and biotechnology. They are used for enantioselective production of alcohols, amines and carboxylic acids. An essential step for the production of biologically active lipases is the folding mediated by specific and unspecific folding mediators. An example for such a folding mediator is the P. Aeruginusa foldase LipH. However, this catalytic folding conventionally takes place for a secreted lipase in form of a periplasmatic folding mediated by said folding mediators.

In a preferred embodiment, the present invention is directed to cells or multitude of cells expressing on their surface chaperon-dependent lipases from the genera Pseudomonas and Burkholderia, and to membrane preparations prepared from such cells and to polypeptides prepared from such cells, multitude of cells or membrane preparations. These enzymes are of particular industrial interests, since they catalyze a wide range of different reactions, both in water and in nonpolar solvents under mild conditions. At the same time, these enzymes are strongly stereoselective.

However, for the correct folding, these lipases require the activity of specific folding mediators, example in form of chaperones. A preferred class of chaperones in the context of the present invention are the "LIF"-proteins (lipase specific proteases). These LIF-proteins represent a unique family of steric chaperones, which are found in the periplasmatic site of the inner membrane of different Gram-negative bacteria.

Yang et al. (2010) expressed chaperone-dependent lipases from Pseudomonas and Burkholderia on the surface of E. Coly. Respective chaperones were coexpressed, however, not by use of an autotransporter-system and also not on the surface of the bacteria. Accordingly, there was no interaction between the chaperone and the lipase and there was certainly no activation of the lipase on the surface of the bacteria. Lee et al. (2005) expressed lipase from Pseudomonas flourescens on the surface of a Pseudomonas-strain, whereby no chaperone-dependent lipase was expressed. Such a non chaperone dependent lipase was also expressed at the surface of the E. coli by Choi et al.

Good results are achieved, when the ratio of activating polypeptide to biologically active polypeptide is between 1:5 and 5:1.

Preferably, the ratio of activation polypeptide to biologically active polypeptide is between 1:1.5 and 1.5:1.

Preferably, the transporter domain of at least one polypeptide is selected from the group comprising Ssp, Ssp-h1, Ssp-h2, PspA, PspB, Ssa1, SphB1, AspA/Na1P, VacA, AIDA.I, lcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protease, App, Hap, rOmpA, rOpmB, ApeE, EstA, Lip-1, McaP, BabA, SabA, AlpA, Aae, NanB, Hly, ClyA, lav, Lpp-OmpA, TraT, Pal, Oprl, and Inp and variants thereof.

Preferably, the cell or multitude of cells described above is a bacterial cell. In a preferred embodiment, the cell or multitude of cells described above comprises E. coli, Pseudomonas and/or Burkholderia.

The object underlying the present invention is also solved by a membrane preparation prepared from a cell or a multitude of cells as described above. In a preferred embodiment, this membrane preparation comprises an active lipase.

The object underlying of the present invention is also solved by a biologically active polypeptide prepared from a cell or multitude of cells described above.

In a preferred embodiment, the biologically active polypeptide comprises a lipase.

The object underlying of the present invention is also solved by use of a cell or multitude of cells described above, a membrane fraction as described above, or a polypeptide as described above for producing a product of a reaction catalyzed by a biologically active polypeptide.

In a preferred embodiment, the cell or multitude of cells, membrane fraction, or polypeptide is used for producing a product of a reaction catalyzed by a lipase.

The problem underlying the present invention is solved by a method for producing the product of a biologically active polypeptide, comprising the steps of providing the cell or multitude of cells described above, the membrane fraction described above, or the biologically active polypeptide described above, optionally removing the activating protein, contacting the cell or multitude of cells, the membrane fraction, or the biologically active polypeptide, with the activating protein optionally being removed, with at least one substrate of the biologically active protein.

The problem underlying the present invention is further solved by use of a cell, or multitude of cells as described above, or the membrane fraction as described above, or the biologically active polypeptide as described above for the production of a product of a biologically active polypeptide.

The present invention is further directed to other aspects. In one further aspect, the present invention is directed to the following embodiments.

Embodiment 1, a nucleic acid encoding a first polypeptide, which shows a biological activity subject to activation by a second polypeptide, wherein the nucleic acid comprises (1) a portion encoding a signal peptide (2) a portion encoding the first polypeptide (3) optionally a portion encoding a protease recognition site (4) optionally a portion encoding a transmembrane linker, and (5) a portion encoding a transporter domain of an autotransporter; wherein at least one of (1)-(5) comprises a heterologous sequence.

Embodiment 2, the nucleic acid according to embodiment 1, wherein the transporter domain of an autotransporter is selected from a group comprising Ssp, Ssp-h1, Ssp-h2, PspA, PspB, Ssa1, SphB1, AspA/Na1P, VacA, AIDA.I, lcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, lgA1 protease, App, Hap, rOmpA, rOpmB, ApeE, EstA, Lip-1, McaP, BabA, SabA, AlpA, Aae, NanB, Hly, ClyA, lav, Lpp-OmpA, TraT, Pal, Oprl, and Inp and variants thereof.

Embodiment 3, a polypeptide comprising the polypeptide encoded by the nucleic acid according to any of embodiments 1 and 2 or a composition comprising said polypeptide, wherein the polypeptide is a polypeptide is a polypeptide expressed on the surface of a cell.

Embodiment 4, a cell comprising or expressing the nucleic acid according to any of embodiments 1 and 2 or the polypeptide according to embodiment 3.

Embodiment 5, a membrane fraction comprising the nucleic acid according to any of embodiments 1 and 2, the polypeptide according to embodiment 3 or the cell according to embodiment 4 or obtainable from the cell according to embodiment 4.

Embodiment 6, the composition according to embodiment 3, the cell according to embodiment 4 or the membrane fraction according to embodiment 5, further comprising the second polypeptide.

Embodiment 7, The composition, the cell or the membrane fraction according to embodiment 6, wherein the second polypeptide is encoded by a nucleic acid comprising (1) a portion encoding a signal peptide (2) a portion encoding the second polypeptide (3) optionally a portion encoding a protease recognition site (4)optionally a portion encoding a transmembrane linker, and (5) a portion encoding a transporter domain on an autotransporter wherein the second polypeptide is a polypeptide expressed on the surface of a cell.

Embodiment 8, the composition, the cell or the membrane fraction according to embodiment 6, wherein the second polypeptide is a polypeptide expressed at a location other than the surface of a cell.

Embodiment 9, the composition, the cell, or the membrane fraction according to embodiment 7, wherein the first and the second polypeptide are expressed on the surface of the same cell.

Embodiment 10, the composition, the cell, or the membrane fraction according to embodiment 7, wherein the first and the second polypeptide are expressed on the surface of different cells.

Embodiment 11, the nucleic acid according to any of embodiments 1 and 2, the polypeptide according to embodiment 3, the composition according to any of embodiments 6 or 7 to 10, the cell according to any of embodiments 4 and 7 to 10, or the membrane fraction according to any of embodiments 5 and 7 to 10, wherein the first polypeptide is activated by a chemical modification catalyzed by the second polypeptide.

Embodiment 12, the nucleic acid according to any of embodiments 1 and 2, the polypeptide according to embodiment 3, the composition according to embodiments 6 or 7 to 10, the cell according to any of embodiments 4 and 7 to 10, or the membrane fraction according to any of embodiments 5 and 7 to 10, wherein the first polypeptide is activated by a conformational change brought about by a chaperone-like activity of the second polypeptide.

Embodiment 13, the nucleic acid, the polypeptide, the composition, the cell or the membrane fraction according to embodiment 12, wherein the first the polypeptide comprises a chaperone-dependent lipase and the second the polypeptide comprises a chaperone that recognizes as its substrate said chaperone-dependent lipase.

Embodiment 14, the nucleic acid, the polypeptide, the composition, the cell or the membrane fraction according to embodiment 13, wherein the first the polypeptide and the second polypeptide are the polypeptides each and independently selected from the group of genera comprising Pseudomonas, Burkholderia and Acetinobacter.

Embodiment 15, a method for producing the product of a first the polypeptide, which shows a biological activity subject to activation by a second polypeptide, comprising the steps a) providing the polypeptide according to any of embodiments 3 and 11 to 14, the composition according to any of embodiments 3 and 7 to 14, the cell according to embodiments 4 and 7 to 14, or the membrane fraction according to any of embodiments 5 and 7 to 14, wherein the composition, the cell or the membrane fraction does not comprise the second the polypeptide, and contacting it with the second polypeptide under conditions compatible with activation of the first polypeptide by the second the polypeptide; or providing the polypeptide according to any of embodiments 11 to 14, the composition according to any of embodiments 6 to 14, the cell according to embodiments 6 to 14 or the membrane fraction according to any of embodiments 5 to 14, wherein the composition, the cell or the membrane fraction comprises the second the polypeptide; b.) (optionally) isolating the first the polypeptide; c.) contacting the composition, cell or membrane fraction provided, or provided and contacted, in step a.), or the first the polypeptide isolated in step b.) with a substrate of the first the polypeptide.

The present invention is directed to a further aspect. This further aspect is directed to the following-embodiments:

Embodiment 1, a nucleic acid molecule comprising the following components (1) a portion encoding a signal peptide (2) a portion encoding a nucleic acid-processing polypeptide (3) optionally a portion encoding a protease recognition site (4) optionally a portion encoding a transmembrane linker, and (5) a portion encoding a transporter domain of an autotransporter.

Embodiment 2, the nucleic acid molecule according to embodiment 1, wherein the nucleic acid processed by the nucleic acid-processing polypeptide is or comprises DNA and/or RNA, each modified or unmodified.

Embodiment 3, the nucleic acid molecule according to embodiment 2, wherein the nucleic acid-processing polypeptide is selected from the group comprising lipase, nucleic acid polymerase, reverse transcriptase, nucleic acid phosphatase, nucleic acid kinase, nucleic acid methylase, topoisomerase and nuclease.

Embodiment 4, the nucleic acid molecule according to embodiment 3, wherein the nucleic acid-processing polypeptide is a nuclease that has specific endonuclease activity.

Embodiment 5, the nucleic acid molecule according to any of embodiments 3 to 4, wherein the nucleic acid-processing polypeptide has type II restriction endonuclease activity.

Embodiment 6, the nucleic acid molecule according to embodiment 5, wherein the nucleic acid-processing polypeptide is selcted from the group of restriction endonucleases comprising Aatll, Acc65l, Accl, Acil Acll, Acul, Afel, Aflll, Afllll, Agel, Ahdl, Alel, Alul, Alwl, AlwNl, Apal, ApaLl, ApeKl, Apol, Ascl, Asel, AsiSl, Aval, Avall, Avrll, BaeGl, Bael, BamHl, Banl, Banll, Bbsl, BbvCl, Bbvl, Bccl, BceAl, Bcgl, Bcll, BciVl, Bfal, BfuAl, BfuCl, Bgll, Bglll, Blpl, BmgBl, Bmrl, Bmtl, Bpml, Bpu10l, BpuEl, BsaAl, BsaBl, BsaHl, Bsal, BsaJl, BsaWl, BsaXl, BseRl, BseYl, Bsgl, BsiEl, BsiHKAl, Bsll, BsiWl., BsmAl, BsmBl, BsnFl, Bsml, BsoBl, Bsp1286l, BspCNl, BspDl, BspEl, BspHl, BspMl, BSpQl, BsrBl, BsrDl, BsrFl, BsrGl, BSrl, BssHll, BssKl, BssSl, BstAPl, BstBl, BstEll, BstNl, BstUl, BstXl, BstYl, BstZ17l, Bsu 36l, Btgl, BtgZl, BtsCl, Btsl, Cac8l, Clal, CspCl, CviAll, CviKI-1, CviQl, Ddel, Dpnl, Dpnll, Dral, Dralll, Drdl, Eael, Eagl, Earl, Ecil Eco53kl, EcoNl, EcoO109l, EcoP15l, EcoRl, EcoRV, Fatl, Faul Fnu4Hl, Fokl, Fsel, Fspl, Haell, Haelll, Hgal, Hhal, Hincll, Hindlll, Hinfl, HinP1l, Hpal, Hpall, Hphl, Hpy166ll, Hpy188l, Hpy188lll, Hpy4V, Hpy99l, HpyCH4lll, HpyCH4lV, HpyCH4V, I-Ceul, I-Scel, Kasl, Kpnl, Mobol, Mboll, Mfel, Mlul, Mlyl, Mmel, Mnll, Mscl, Msel, Msll, MSpA1l, Mspl, Mwol, Nael, Narl, Nb.BbvCl, Nb.BsrDl, Nb.Bsrnl, Nb.Btsl, Ncil, Ncol, Ndel, NgoMlV, Nhel, Nlalll, NlalV, NmeAlll, NotI Nrul, Nsil, Nspl, Nt.Alwl, Nt.BbvCl, Nt.BsmAl, Nt.BspQl, Nt.BstNBl, Nt.CviPll, Pacl, PaeR7l, Pcil PflFl, PflMl, Phol, Plel, PI-Pspl, PI-Scel, Pmel, Pmll, PpuMl, PshAl, Psil, PspGl, PspOMl, PspXl, Pstl, Pvul, Pvull, Rsal, Rsrll, Sacl, Sacll, Sall, Sapl, Sau3Al, sau96l, Sbfl, Scal, ScrFl, SexAl, SfaNl, Sfcl, Sfil, Sfol, SgrAl, Smal, Smll, SnaBl, Spel, Sphl, Sspl, Stul, StyD4l, Styl, Swal, Taql, Tfil, Tlil, Tsel, Tsp45l, Tsp509l, TspMl, TspRl, Tth111l, Xbal, Xcml, Xhol, Xmal, Xmnl and Zral and variants thereof.

Embodiment 7, the nucleic acid molecule according to any of embodiments 1 to 6, wherein the transporter domain of an autotransporter is selcted from a group comrpsing MisL, Ssp, Ssp-h1, Ssp-h2, PspA, PspB, Ssa1, SphB1, AspA/Na1P, VacA, AIDA.I, lcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protease, App, Hap, rOmpA, rOpmB, ApeE, EstA, Lip-1, McaP, BabA, SabA, AlpA, Aae, NanB, Hly, ClyA, lav, Lpp-OmpA, BtuB, TraT, Pal, Oprl, and Inp and variants thereof.

Embodiment 8, a polypeptide encoded by the nucleic acid molecule according to any of embodiments 1 to 7.

Embodiment 9, a cell expressing on its surface the polypeptide according to embodiment 8 or is a cell transformed using the nucleic acid molecule according of any of embodiments 1 to 7.

Embodiment 10, the cell according to embodiment 9, wherein the cell expresses on its surface more than one polypeptide according to embodiment 8 or is a cell transformed using more than one nucleic acid molecule according to any of embodiments 1 to 7.

Embodiment 11, the cell according to embodiment 10, wherein the more than one nucleic acid molecules encode, as portion (2) as specified in embodiment 1, or wherein the cell expresses on its surface at least two nucleic acid-processing polypeptides of different activities and/or specificities.

Embodiment 12, the cell according to embodiment 11, herein the more than one nucleic acid molecules encode, as portion (2) as specified in embodiment 1, or wherein the cell expresses on its surface at least two restriction endonucleases of different activities and/ or specificities.

Embodiment 13, a membrane fraction obtainable from the cell according to any of embodiments 9 to 12.

Embodiment 14, a method for processing a substrate nucleic acid, comprising the steps a) contacting a substrate nucleic acid with the polypeptide according to embodiment 8, the cell according to any of claims 9 to 12 or the membrane preparation according to claim 13 under conditions compatible with processing of the nucleic acid, (b) optionally removing the membrane preparation.

Embodiment 15, use of the nucleic acid according to any of embodiments 1 to 7, the polypeptide according to embodiment 8, the cell according to any of embodiments 9 to 12 or the membrane preparation according to embodiment 13 for producing the product of a nucleic acid-processing polypeptide.

The present invention is also directed to a further aspect. This further aspect is directed to the following embodiments:
Embodiment 1, a nucleic acid molecule comprising the following components: (1) a portion encoding a signal peptide (2) a portion comprising a heterologous protease to be displayed using autodispla (3) optionally a portion encoding a protease recognition site (4) optionally a portion encoding a transmembrane-linker, and (5) a portion encoding a transporter domain of an autotransporter.
Embodiment 2, the nucleic acid molecule according to embodiment 1, wherein the protease is a cysteine protease.
Embodiment 3, the nucleic acid molecule according to any of embodiments 1 to 2, wherein the protease is selected from the group comprising (CDR... "hinzufügen?")
Embodiment 4, the nucleic acid molecule according to any of embodiments 1 to 3, wherein the protease is a cytosolic protease.
Embodiment 5, the nucleic acid molecule according to any of embodiments 1 to 4, wherein the transporter domain of an autotransporter is selcted from a group comrpsing MisL, Ssp, Ssp-h1, Ssp-h2, PspA, PspB, Ssa1, SphB1, AspA/Na1P, VacA, AIDA.l, lcsA, MisL, TibA, Ag43, ShdA, AutA, Tsh, SepA, EspC, EspP, Pet, Pic, SigA, Sat, Vat, EpeA, EatA, Espl, EaaA, EaaC, Pertactin, BrkA, Tef, Vag8, PmpD, Pmp20, Pmp21, IgA1 protease, App, Hap, rOmpA, rOpmB, ApeE, EstA, Lip-1, McaP, BabA, SabA, AlpA, Aae, NanB, Hly, ClyA, lav, Lpp-OmpA, BtuB, TraT, Pal, Oprl, and Inp and variants thereof.
Embodiment 6, a polypeptide encoded by a nucleic acid molecule according to any of embodiments 1 to 5.
Embodiment 7, a cell expressing on its surface a polypeptide according to embodiment 6 or transformed using a nucleic acid molecule according of any embodiments 1 to 5.
Embodiment 8, a membrane fraction obtainable from a cell according to embodiment 7.
Embodiment 9, the membrane preparation according to embodiment 8, wherein the membrane preparation comprises solubilized membranes.
Embodiment 10, composition a membrane preparation according to any of embodiments 6 to 7, a polypeptide according to embedment 6 and / or a cell according to embodiment 7, as well as at least one specific protease inhibitor.
Embodiment 11, a method for cleaving polypeptide featuring a protease cleavage site using this protease cleavage site, comprising the step a) contacting a protease substrate and a polypeptide according to embodiment 6, the membrane preparation according to any of embodiments 8 to 9, the cell according to embodiment 7 and / or the composition according to embodiment 10.
Embodiment 12, a method according to embodiment 11, further comprising the step b) removing the membrane preparation.
Embodiment 13, the method according to any of embodiments 11 to 12, wherein removing the membrane preparation is performed using centrifugation, tangential flow preparation electrophoresis or filtration.
Embodiment 14, the method according to any of embodiments 11 to 13, wherein the protease substrate is a fusion protein comprising a target protein and at least one tag.
Embodiment 15, the method according to any of embodiments 11 to 14, wherein the protease substrate comprises a cleavage site of a protease between the target protein and the at least one tag.
Embodiment 16, reaction vessel comprising an immobilized phase comprising the membrane preparation according to any of embodiments 8 to 9, the polypeptide according to embodiment 6 or the cell according to embodiment 7.
Embodiment 17, a use of the cell according to embodiment 7 or the membrane preparation according to any of embodiments 8 to 9 or the polypeptide according to embodiment 6 or the composition according to embodiment 10 for preparing a protease.

## Claims

1. Cell or multitude of cells in form of
a cell expressing on its surface an activating polypeptide and a biologically active polypeptide, which biologically active polypeptide shows a biological activity subject to activation by the activating polypeptide,
wherein at least one of the activating polypeptide and/or the biologically active polypeptide is encoded by a nucleic acid comprising
(1) a portion encoding a signal peptide
(2) a portion encoding the biologically active polypeptide and/or the activating polypeptide
(3) optionally a portion encoding a protease recognition site
(4) optionally a portion encoding a transmembrane linker, and
(5) optionally a portion encoding a transporter domain of an autotransporter;
or
a multitude of cells comprising a first cell expressing on its surface an activating polypeptide and a second cell expressing on its surface a biologically active polypeptide,
wherein at least one of the activating polypeptide and/or the biologically active polypeptide is encoded by a nucleic acid comprising
(1) a portion encoding a signal peptide
(2) a portion encoding the biologically active polypeptide and/or the activating polypeptide
(3) optionally a portion encoding a protease recognition site
(4) optionally a portion encoding a transmembrane linker, and
(5) optionally a portion encoding a transporter domain on an autotransporter

2. A cell according to claim 1, wherein the cell expresses on its surface a fusion polypeptide comprising both the activating polypeptide and the biologically active polypeptide.

3. Cell or multitude of cells according to any of the preceding claims, wherein the biologically active polypeptide comprises a chaperone-dependent lipase and the activating polypeptide comprises a chaperone that recognizes as its substrate said chaperone-dependent lipase.

4. Cell or multitude of cells according to any of the preceding claims, wherein the biologically active polypeptide and the activating polypeptide are independently selected from the polypeptides derived from the genera Pseudomonas, Burkholderia, Chromobacterium, Ralstonia, Acinetobacter, Vibrio and Xylella.

5. Cell or multitude of cells according to any of the preceeding claims, wherein the biologically active polypeptide is derived from Pseudomonas aeruginosa (accession no. D50587), Pseudomonas fragi (accession no. X14033), Pseudomonas luteola (accession no. AF050153), Pseudomonas wisconsinensis (accession no. U88907), Pseudomonas alcaligenes (accession no. CFR 708) Burkholderia cepacia (accession no. M58494), Burkholderia glumae (accession no. X70354), Chromobacterium viscosum (accession no. Q05489), Ralstonia metallidurans, Ralstonia solanacearum (accession no. AL 646081), Acinetobacter calcoaceticus (accession no. X80800), Vibrio cholerae (accession no. X16945), Vibrio vulnificus, Xylella fastidosa and variants thereof.

6. Cell or multitude of cells according to any of the preceding claims, wherein the activating polypeptide is a foldase.

7. Cell or multitude of cells according to any of the preceding claims, wherein the ratio of activating polypeptide to biologically active polypeptide is between 1:5 and 5:1.

8. Cell or multitude of cells according to any of the preceding claims, wherein the ratio of activating polypeptide to biologically active polypeptide is between 1:1.5 and 1.5:1.

9. Cell or multitude of cells according to any of the preceding claims, wherein the transporter domain of at least one polypeptide is selected from the group comprising Ssp, Ssp-h1, Ssp-h2, PspA, PspB, Ssa1, SphB1, AspA/Na1P, VacA, AIDA.I, IcsA, MisL, TibA, Ag43, ShdA, AutA, Tsh, SepA, EspC, EspP, Pet, Pic, SigA, Sat, Vat, EpeA, EatA, EspI, EaaA, EaaC, Partactin, BrkA, Tef, Vag8, PmpD, Pmp20, Pmp21, IgA1 protease, App, Hap, rOmpA, rOpmB, ApeE, EstA, Lip-1, McaP, BabA, SabA, AlpA, Aae, NanB, Hly, ClyA, lav, Lpp-OmpA, TraT, Pal, Oprl, and INp and variants thereof.

10. Membrane preparation prepared from a cell or a multitude of cells according to any of the preceding claims.

11. Membrane preparation according to claim 10, comprising an active lipase.

12. Biologically active polypeptide, prepared from a cell or multitude of cells according to any of the claims 1 to 9 or a membrane preparation according to claims 10 or 11.

13. Biologically active polypeptide according to claim 12, comprising a lipase.

14. Use of a cell or a multitude of cells according any of the claims 1 to 9, a membrane fraction according to claims 10 or 11 or a polypeptide according to claims 10 or 11 or a polypeptide according to claims 12 or 13 for producing a product of a reaction catalyzed by a biologically active polypeptide.

15. Use according to claim 14, for producing a product of a reaction catalyzed by a lipase.
